(19) Europäisches Patentamt
European Patent Office
Office européen des brevets

(11) **EP 3 274 694 B1**

(12) **FASCICULE DE BREVET EUROPEEN**

(45) Date de publication et mention
de la délivrance du brevet:
**18.12.2019 Bulletin 2019/51**

(21) Numéro de dépôt: **16714496.3**

(22) Date de dépôt: **23.03.2016**

(51) Int Cl.:
*G01N 21/47* (2006.01)   *G01N 21/45* (2006.01)
*G01N 21/51* (2006.01)   *G03H 1/04* (2006.01)
*G03H 1/08* (2006.01)   *G01N 15/14* (2006.01)
*G06K 9/00* (2006.01)   *G06T 7/00* (2017.01)
*C12M 1/34* (2006.01)   *G01N 33/483* (2006.01)
*G01N 15/00* (2006.01)   *G01N 15/10* (2006.01)
*G01N 21/17* (2006.01)   *G03H 1/00* (2006.01)

(86) Numéro de dépôt international:
**PCT/FR2016/050644**

(87) Numéro de publication internationale:
**WO 2016/151249 (29.09.2016 Gazette 2016/39)**

(54) **PROCÉDÉ DE DÉTERMINATION DE L'ÉTAT D'UNE CELLULE**

VERFAHREN ZUR BESTIMMUNG DES ZUSTANDES EINER ZELLE

METHOD FOR DETERMINING THE STATE OF A CELL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.03.2015 FR 1552445**

(43) Date de publication de la demande:
**31.01.2018 Bulletin 2018/05**

(73) Titulaires:
• **Commissariat à l'Energie Atomique et aux Energies Alternatives**
  **75015 Paris (FR)**
• **Iprasense SAS**
  **34830 Clapiers (FR)**

(72) Inventeurs:
• **ALLIER, Cédric**
  **38000 Grenoble (FR)**
• **ESTEBAN, Geoffrey**
  **34820 Teyran (FR)**
• **HERVE, Lionel**
  **38700 Corenc (FR)**

(74) Mandataire: **GIE Innovation Competence Group**
  **310, avenue Berthelot**
  **69372 Lyon Cedex 08 (FR)**

(56) Documents cités:
WO-A1-2014/012031    US-A1- 2012 148 141
US-A1- 2014 133 702

• S. VINJIMORE KESAVAN ET AL: "High-throughput monitoring of major cell functions by means of lensfree video microscopy", SCIENTIFIC REPORTS, vol. 4, 6 août 2014 (2014-08-06), XP055250876, DOI: 10.1038/srep05942
• KAREN M. MOLONY ET AL: "Segmentation and visualization of digital in-line holographic microscopy of three-dimensional scenes using reconstructed intensity images", MEDICAL IMAGING 2002: PACS AND INTEGRATED MEDICAL INFORMATION SYSTEMS: DESIGN AND EVALUATION, vol. 7443, 20 août 2009 (2009-08-20), page 74431F, XP055251117, 1000 20th St. Bellingham WA 98225-6705 USA ISSN: 0277-786X, DOI: 10.1117/12.826832 ISBN: 978-1-5106-0167-3

• MISSAN SERGEY ET AL: "Using digital inline holographic microscopy and quantitative phase contrast imaging to assess viability of cultured mammalian cells", PROGRESS IN BIOMEDICAL OPTICS AND IMAGING, SPIE - INTERNATIONAL SOCIETY FOR OPTICAL ENGINEERING, BELLINGHAM, WA, US, vol. 9336, 11 mars 2015 (2015-03-11), pages 93361X-93361X, XP060049373, ISSN: 1605-7422, DOI: 10.1117/12.2078284 ISBN: 978-1-5106-0027-0

• YUNXIN WANG ET AL: "Non-invasive monitoring for living cell culture with lensless Fourier transform digital holography microscopy", MEDICAL IMAGING 2002: PACS AND INTEGRATED MEDICAL INFORMATION SYSTEMS: DESIGN AND EVALUATION, vol. 7791, 2 août 2010 (2010-08-02), pages 77910E-77910E-8, XP055250808, 1000 20th St. Bellingham WA 98225-6705 USA ISSN: 0277-786X, DOI: 10.1117/12.860164 ISBN: 978-1-5106-0167-3

**Description**

**DOMAINE TECHNIQUE**

L'invention se situe dans le domaine de l'analyse de cellules, et plus précisément dans le contrôle de la prolifération de cellules, dans des incubateurs ou des réacteurs biologiques.

**ART ANTERIEUR**

Le contrôle du développement de cellules dans des incubateurs ou dans des réacteurs biologiques est une étape essentielle dans le processus de productions de cellules. Dans ces applications, les cellules sont disposées dans un milieu de culture, propice à leur développement.

On contrôle régulièrement leur nombre ainsi que leur état, et en particulier le fait qu'elles soient vivantes ou mortes. Ces opérations de contrôles nécessitent l'emploi d'un microscope, les cellules étant préalablement marquées à l'aide d'un label fluorescent ou d'un chromophore, le niveau de fluorescence de cellules variant selon qu'elles soient vivantes ou mortes. Un tel procédé comporte certains inconvénients : tout d'abord, il nécessite l'utilisation d'un microscope, équipement couteux et encombrant. De plus, le champ d'observation étant faible, l'analyse d'un échantillon spatialement étendu requiert du temps car il faut déplacer l'échantillon devant le microscope. Par ailleurs, le marquage à l'aide d'un label fluorescent ou du chromophore peut avoir des conséquences sur le développement des cellules.

Un des axes de recherche est l'utilisation de méthodes optiques simples, telle l'imagerie sans lentille. L'observation de particules biologiques par imagerie sans lentille connaît un certain développement depuis la fin des années 2000. Cette technique consiste à intercaler un échantillon entre une source de lumière et un photodétecteur matriciel, ou capteur d'image. L'image recueillie sur le photodétecteur est formée par des interférences entre l'onde incidente, produite par la source de lumière et l'onde diffractée par les particules composant l'échantillon. Cette image est fréquemment désignée par le terme « hologramme ». Ainsi, pour chaque particule, on peut enregistrer au niveau du capteur un motif de diffraction, ou figure de diffraction, qui lui est propre. Appliquée à des échantillons biologiques, cette technique a été décrite dans le document WO2008090330. Il est alors possible d'effectuer une analyse simple de chaque particule, en comparant la figure de diffraction qu'elle engendre avec des figures de diffraction préalablement établies, correspondant à des particules connues. Mais cette méthode peut trouver des limites lorsque la concentration des particules augmente.

Il est possible d'appliquer des traitements mathématiques dits de reconstruction holographique numérique afin d'établir une image, dite image complexe, de chaque particule présente dans l'échantillon. Cette méthode consiste à retro-propager l'onde lumineuse dans le plan objet, dans lequel sont situées les particules, ce dernier étant placé à une distance connue de l'imageur. Des applications à la caractérisation de cellules sur la base d'une image complexe reconstruite a été décrite dans les documents US2012/0148141, ou WO2014/012031, les cellules étant des spermatozoïdes. Mais ces méthodes se limitent à estimer des propriétés desdites cellules, ainsi que leur trajectoire, à partir de l'image complexe reconstruite. Or, le recours à une image complexe d'un échantillon peut être insuffisant pour caractériser une particule.

On recherche donc un procédé d'observation de cellules, et en particulier un moyen pour discriminer des cellules vivantes ou mortes, simple, peu onéreux, fiable, sans marquage et offrant un champ d'observation étendu.

**EXPOSE DE L'INVENTION**

L'invention répond à ce problème, en proposant un procédé pour déterminer l'état d'une cellule ladite cellule étant placée dans un échantillon, le procédé comportant les étapes suivantes :

- illumination dudit échantillon à l'aide d'une source de lumière, la source de lumière produisant une onde lumineuse incidente se propageant vers l'échantillon selon un axe de propagation,
- acquisition, à l'aide d'un photodétecteur matriciel, d'une image de l'échantillon, l'échantillon étant disposé entre ladite source de lumière et ledit photodétecteur matriciel, de telle sorte que le photodétecteur matriciel est exposé à une onde lumineuse comprenant des interférences entre l'onde lumineuse incidente et une onde de diffraction produite par chaque cellule ;
- détermination d'une position de ladite cellule dans un plan parallèle à un plan de détection selon lequel s'étend le photodétecteur matriciel ; le procédé étant caractérisé en ce qu'il comprend les étapes suivantes :

    - application d'un algorithme de reconstruction numérique à ladite image acquise, de façon à déterminer au moins une grandeur caractéristique de l'onde lumineuse à laquelle est exposé le photodétecteur matriciel, à ladite position, à une pluralité de distances de ce dernier, dites distances de reconstruction, selon ledit axe de propagation ;

- classification de la cellule en fonction d'un profil représentant une évolution de ladite grandeur caractéristique selon l'axe de propagation, la classification permettant de déterminer l'état de ladite cellule parmi des états prédéterminés.

Le profil est défini en fonction de la valeur de la grandeur caractéristique déterminé à ladite pluralité de distances.

En particulier, les états prédéterminés peuvent comprendre un état vivant et un état mort. Le procédé est alors apte à classifier une cellule examinée et déterminer si elle est morte ou vivante.

Par application d'un algorithme de reconstruction numérique, on entend l'application d'un opérateur de propagation à une image, généralement sous la forme d'un produit de convolution.

Chaque grandeur caractéristique est notamment obtenue en estimant, à ladite distance de reconstruction, une expression complexe de l'onde lumineuse à laquelle est exposé le photodétecteur matriciel. La grandeur caractéristique peut s'obtenir à partir du module ou de l'argument de ladite expression complexe.

La classification peut être réalisée en comparant ladite évolution de ladite grandeur caractéristique à des profils types prédéterminés.

Selon un mode de réalisation, le procédé comporte :

- la détermination d'une image complexe, dite image complexe de référence, par application d'un algorithme de reconstruction numérique à l'image acquise par le photodétecteur matriciel ;
- à partir de ladite image complexe de référence, l'estimation d'au moins une grandeur caractéristique de l'onde lumineuse à laquelle est exposé le photodétecteur matriciel, à une pluralité de distances de reconstruction de ce dernier.

Le procédé peut alors comporter :

- l'application d'un opérateur de propagation à l'image complexe de référence, de façon à calculer des images complexes dites secondaires, selon une pluralité de distances du plan de reconstruction ou du plan selon lequel s'étend le photodétecteur matriciel ;
- la détermination d'une grandeur caractéristique à chacune desdites distances, à partir de chaque image complexe secondaire.

L'image complexe de référence peut être une image complexe formée dans un plan de reconstruction, distant du plan de l'échantillon. Il peut également s'agir d'une image complexe formée dans le plan de détection.

Le procédé peut comprendre une étape de reconstruction d'une image de ladite grandeur caractéristique selon un plan parallèle au plan de détection, et à ladite distance de reconstruction, la valeur de ladite grandeur caractéristique à ladite position de la cellule, et à ladite distance de reconstruction, étant déterminée en fonction de cette image.

La position de chaque cellule, dans un plan parallèle au plan de détection, peut être déterminée à l'aide de l'image acquise par le photodétecteur matriciel ou à l'aide d'une image reconstruite décrite dans le paragraphe précédent.

La source de lumière peut être une source spatialement cohérente. Il peut en particulier s'agir d'une diode électroluminescente. La source de lumière peut également être temporellement cohérente ; il peut en particulier s'agir d'une diode laser.

Le photodétecteur matriciel, ou capteur d'image, comporte une matrice de pixels, aptes à collecter l'onde lumineuse à laquelle est exposé le photodétecteur. La distance entre les pixels et l'échantillon peut varier entre 50 $\mu$m et 2 cm, et de préférence entre 100 $\mu$m et 5 mm. De préférence l'échantillon n'est pas disposé au contact direct des pixels du photodétecteur matriciel.

De préférence, aucune optique de grossissement n'est disposée entre l'échantillon et le photodétecteur matriciel.

Un objet de l'invention est également un dispositif pour discriminer une cellule vivante d'une cellule morte, ladite cellule étant disposée dans un échantillon, le dispositif comprenant :

- une source de lumière agencée pour produire une onde lumineuse incidente selon un axe de propagation, en direction dudit échantillon,
- un photodétecteur matriciel, agencé pour acquérir une image de l'échantillon, en étant exposé à une onde lumineuse, résultant de l'interférence entre ladite onde lumineuse incidente et une onde de diffraction formée par ladite cellule,
- un support, pour maintenir l'échantillon entre ladite source de lumière et le photodétecteur matriciel,
- -un processeur configuré pour mettre en œuvre l'étape suivante :

- détermination d'une position de ladite cellule dans un plan parallèle à un plan de détection, selon lequel s'étend le photodétecteur matriciel, le dispositif étant caractérisé en ce que ledit processeur est configuré pour mettre en œuvre les étapes suivantes :

- application d'un algorithme de reconstruction numérique à ladite image acquise, de façon à déterminer au moins une grandeur caractéristique de l'onde lumineuse à laquelle est exposé le photodétecteur matriciel, à ladite position, à une pluralité de distances de ce dernier, dites distances de reconstruction, selon l'axe de propagation,
- classification de ladite cellule en fonction d'un profil représentant l'évolution de la ladite grandeur caractéristique selon l'axe de propagation, la classification étant apte à déterminer l'état de la cellule parmi des états prédéterminés.

Le processeur peut être un microprocesseur, relié à une mémoire programmable, comportant une séquence d'instructions pour la réalisation des étapes décrites dans cette description.

De préférence, le dispositif ne comporte pas d'optique de grossissement entre le photodétecteur et l'échantillon analysé.

L'échantillon peut être disposé dans une enceinte transparente, placée entre le photodétecteur et la source de lumière.

Un objet de l'invention est également un incubateur, destiné à la croissance de cellules, comprenant un dispositif tel que préalablement décrit.

## FIGURES

La figure 1 représente le dispositif selon un mode de réalisation de l'invention.

La figure 2 représente une image acquise par le photodétecteur selon un premier exemple de réalisation.

Les figures 3A à 3C représentent, pour un premier exemple de réalisation, des images de la phase de l'onde lumineuse incidente au détecteur, ces images étant issues d'une reconstruction holographique à trois distances de reconstruction différentes.

Les figures 4A et 4B représentent respectivement, pour un premier exemple de réalisation, le profil de la phase et le profil d'une autre grandeur caractéristique, dite amplitude complémentaire, le long de l'axe de propagation, et cela pour 5 cellules.

Les figures 5A et 5B représentent respectivement, pour un deuxième exemple de réalisation, le profil de la phase et le profil d'une autre grandeur caractéristique, dite amplitude complémentaire, le long de l'axe de propagation, et cela pour une pluralité de cellules.

Les figures 6A et 6B représentent respectivement, pour un troisième exemple de réalisation, le profil de la phase et le profil d'une autre grandeur caractéristique, dite amplitude complémentaire, le long de l'axe de propagation, et cela pour une pluralité de cellules.

La figure 7 représente, en relation avec ce troisième exemple de réalisation, le profil d'une grandeur composite combinant la phase et l'absorption le long de l'axe de propagation pour une pluralité de cellules.

La figure 8A illustre les principales étapes d'un procédé permettant le calcul d'une image complexe d'un échantillon dans un plan de reconstruction.

Les figures 8B, 8C, 8D, 8E et 8F représentent respectivement :

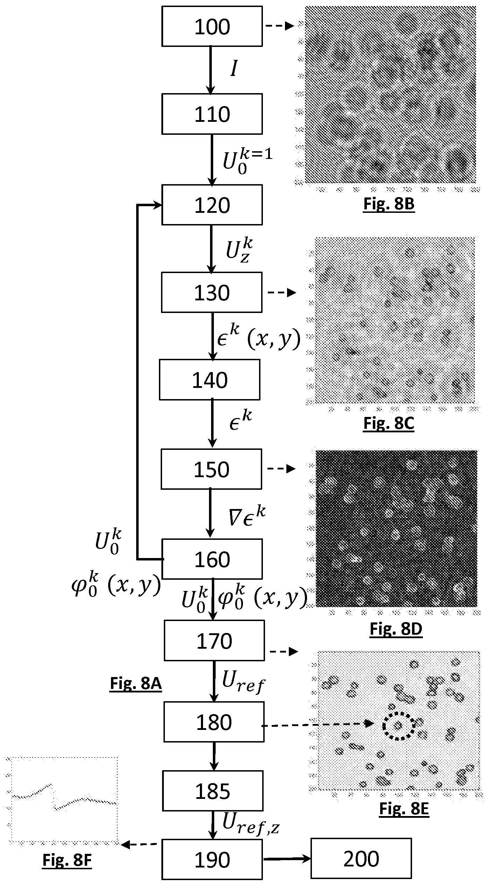

- une image acquise par le photodétecteur matriciel, également désignée par le terme « hologramme » ;
- une image reconstruite dans un plan de reconstruction lors d'une première itération du procédé représenté sur la figure 8A ;
- une image représentant une grandeur associée à chaque pixel de l'image représentée sur la figure 8C ;
- une représentation d'une image, dite image complexe de référence, reconstruite après plusieurs itérations du procédé représenté sur la figure 8A.
- un profil obtenu sur la base d'images complexes secondaires formées à partir de l'image complexe de référence.

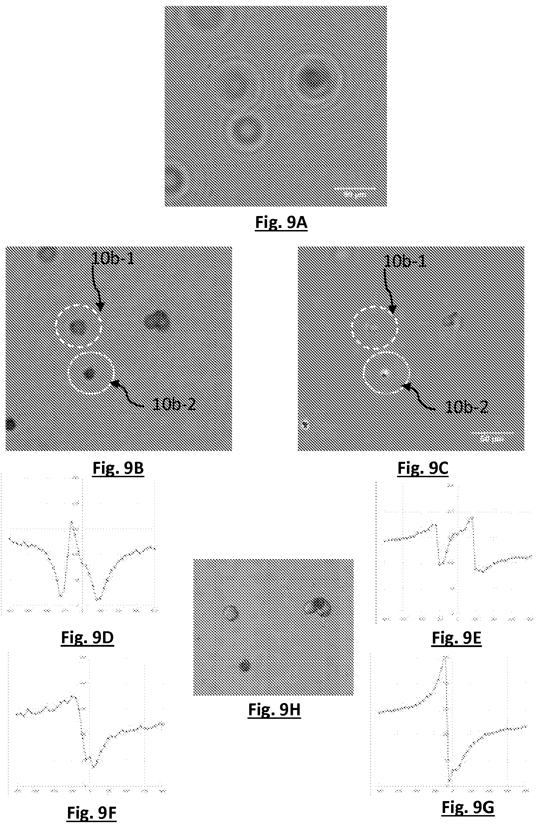

La figure 9A est un hologramme acquis par un capteur d'image, l'échantillon comportant des cellules dispersées dans une solution aqueuse. Les figures 9B et 9C représentent respectivement le module et la phase d'une image complexe, dite de référence, cette image complexe étant formée dans un plan de reconstruction. Les figures 9D et 9E sont des profils représentant respectivement une évolution du module et de la phase de l'onde lumineuse auquel le capteur d'image est exposé, le long d'un axe de propagation passant par une première cellule. Les figures 9F et 9G sont des profils représentant respectivement une évolution du module et de la phase de l'onde lumineuse auquel le capteur d'image est exposé, le long d'un axe de propagation passant par une deuxième cellule. La figure 9H est une image au microscope de l'échantillon observé.

## EXPOSE DE MODES DE REALISATION PARTICULIERS

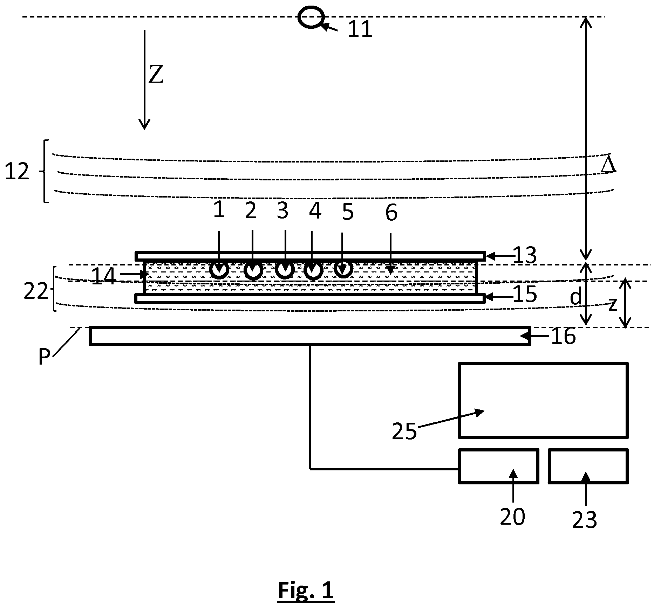

La figure 1 représente un exemple de dispositif selon un mode de réalisation de l'invention. Une source de lumière 11 est apte à produire une onde lumineuse 12, dite onde lumineuse incidente, en direction d'un échantillon 14, selon un axe de propagation Z. L'échantillon 14 comporte un milieu de culture 6 ainsi que des cellules 1, 2, 3, 4, 5 dont on souhaite déterminer l'état, et en particulier si elles sont vivantes ou mortes.

La distance Δ entre la source de lumière et l'échantillon est de préférence supérieure à 1 cm. Elle est de préférence comprise entre 2 et 10 cm, typiquement 5cm. De préférence, la source de lumière, vue par l'échantillon, est considérée comme ponctuelle. Cela signifie que son diamètre (ou sa diagonale) doivent être inférieure au cinquième, mieux au dixième de la distance entre l'échantillon et la source de lumière. Ainsi, la lumière parvient à l'échantillon sous la forme d'ondes planes, ou pouvant être considérées comme telles.

La source de lumière 11 peut être ponctuelle, ou être associée à un diaphragme, non représenté sur la figure 1, de façon à apparaître ponctuelle. L'ouverture du diaphragme est typiquement comprise entre 50 $\mu$m et 1mm, de préférence 50 $\mu$m et 500 $\mu$m.

Le diaphragme peut être remplacé par une fibre optique, dont une première extrémité est placée face à une source lumineuse, et dont une deuxième extrémité est placée face à l'échantillon. Dans ce cas, ladite deuxième extrémité peut être assimilée à une source de lumière ponctuelle 11.

L'échantillon 14 est délimité par une enceinte, comportant un fond 15 et un couvercle 13. Les parois latérales de l'enceinte ne sont pas représentées. Typiquement une enceinte est une boite de Pétri ou le puits d'une plaque à puits. Dans l'exemple considéré, le fond 15 et le couvercle 13 sont constitués de 2 lames transparentes distantes de 100 $\mu$m. La distance d entre les cellules 1,2,3,4,5 et le photodétecteur 16 est égale à 3450 $\mu$m.

D'une façon générale, l'épaisseur de l'enceinte, selon l'axe de propagation Z, est de préférence inférieure à quelques cm, par exemple inférieure à 5 cm, voire inférieure à 1 cm.

La source de lumière 11 peut être temporellement cohérente mais cela n'est pas nécessaire.

Dans cet exemple, la source de lumière est une diode électroluminescente OSRAM, de référence LA E67B-U2AA-24-1. Elle est située à une distance Δ égale 5 cm de l'échantillon.

L'échantillon 14 est disposé entre la source de lumière 11 et un photodétecteur matriciel 16. Ce dernier s'étend selon un plan de détection P, de préférence parallèlement, ou sensiblement parallèlement au fond 15 de l'enceinte délimitant l'échantillon. Le plan de détection P s'étend de préférence perpendiculairement à l'axe de propagation Z.

Le terme sensiblement parallèlement signifie que les deux éléments peuvent ne pas être rigoureusement parallèles, une tolérance angulaire de quelques degrés, inférieure à 10° étant admise.

De préférence, la source de lumière est de faible largeur spectrale, par exemple inférieure à 200 nm, voire 100 nm ou même 25 nm. Le terme largeur spectrale désigne la largeur à mi-hauteur du pic d'émission de la source de lumière.

Le photodétecteur 16 peut être un photodétecteur matriciel, comportant une matrice de pixels, de type CCD ou un CMOS. Les CMOS sont les photodétecteurs préférés car la taille des pixels est plus faible, ce qui permet d'acquérir des images dont la résolution spatiale est plus favorable. Dans cet exemple, le détecteur est un capteur 12 bits APTINA, référence MT9P031. Il s'agit d'un capteur CMOS RGB, dont le pas inter-pixels est de 2,2 $\mu$m. La surface utile du photodétecteur est de 5,7 x 4,3 mm². Les photodétecteurs dont le pas inter pixel est inférieur à 3 $\mu$m sont préférés, car ils permettent d'obtenir des images avec une résolution spatiale satisfaisante.

De préférence, le photodétecteur comprend une matrice de pixels, au-dessus de laquelle est disposée une fenêtre de protection transparente. La distance entre la matrice de pixels et la fenêtre de protection est généralement comprise entre quelques dizaines de $\mu$m à 150 ou 200 $\mu$m.

D'une manière générale, et cela quel que soit le mode de réalisation, la distance d entre une particule et les pixels du photodétecteur est préférentiellement comprise entre 50 $\mu$m et 2 cm, de préférence comprise entre 100 $\mu$m et 2 mm.

On remarque l'absence d'optique de grossissement entre le photodétecteur matriciel 16 et l'échantillon 14. Cela n'empêche pas la présence éventuelle de microlentilles de focalisation au niveau de chaque pixel du photodétecteur 16.

Dans ce premier exemple, le milieu de culture est un milieu de type DMEM (Dubelcco's Modified Eagle's Médium). L'échantillon contient également des cellules de type Fibroblaste 3T3, dont la concentration est d'environ 0,5 10⁶ cellules par ml.

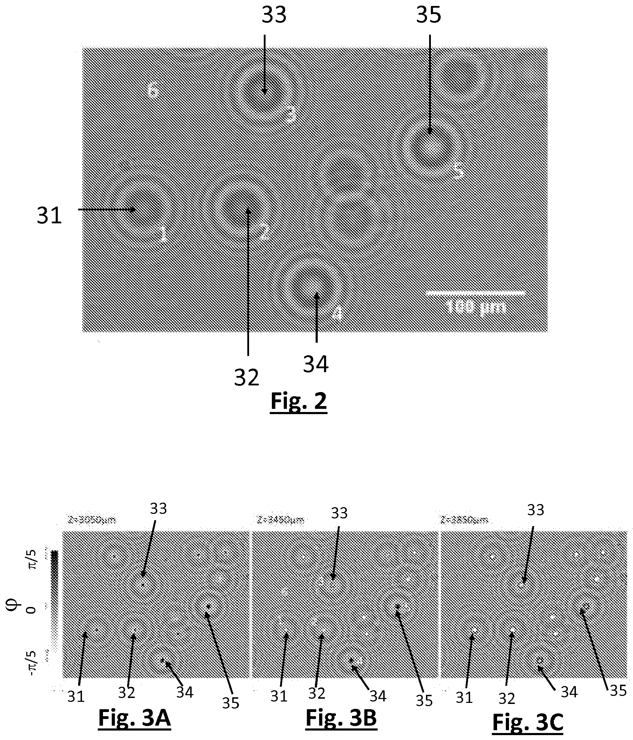

La figure 2 représente une image obtenue par le photodétecteur 16. Cette figure représente une figure de diffraction globale, dans laquelle on distingue des figures de diffraction élémentaires 31, 32, 33, 34, 35, chaque figure de diffraction élémentaire étant respectivement associée aux cellules 1, 2, 3, 4 et 5. Chaque figure de diffraction élémentaire comprend une zone centrale en forme de disque, autour de laquelle s'étendent des anneaux concentriques, alternativement sombres et clairs. La zone repérée par le chiffre 6 correspond à une zone de fond, ne comportant pas de cellule.

Chaque figure de diffraction élémentaire (31,..35) est formée par l'interférence entre l'onde lumineuse incidente 12 produite par la source 11, en amont de l'échantillon, et d'une onde de diffraction de cette onde incidente produite par chaque cellule (1,...,5). Ainsi, le photodétecteur 16 est exposé à une onde lumineuse 22 formée par la superposition :

- de l'onde lumineuse incidente 12 émis par la source 11, en amont de l'échantillon 14,
- de l'onde de diffraction produite par chacune des cellules 1,..,5 ou autres éléments diffractants, présents dans l'échantillon.

Un processeur 20, reçoit les images du photodétecteur matriciel 16 et effectue une reconstruction de grandeurs caractéristiques de l'onde lumineuse 22 à laquelle est exposé le photodétecteur, le long de l'axe de propagation Z. La reconstruction est notamment réalisée entre le photodétecteur et l'échantillon observé. Le processeur 20 peut être apte à exécuter une séquence d'instructions stockées dans une mémoire, pour la mise en œuvre des étapes du procédé d'identification. Le microprocesseur 20 est relié à une mémoire 23 apte à stocker des instructions pour la mise en œuvre des étapes de calcul décrites dans cette demande. Il peut être relié à un écran 25. Le processeur peut-être un microprocesseur, ou tout autre calculateur électronique apte à traiter les images fournies par le photodétecteur matriciel, pour executer une ou plusieurs étapes décrites dans cette description.

L'image représentée sur la figure 2 correspond à la distribution d'intensité I(x,y), x et y désignant les coordonnées dans le plan P de détection précédemment décrit.

Selon les principes bien connus de la reconstruction holographique numérique, décrits dans la publication Ryle et al, « Digital in-line holography of biological specimens », Proc. Of SPIE Vol.6311 (2006), en effectuant un produit de convolution entre l'intensité I(x,y) mesurée par le photodétecteur, et un opérateur de propagation h(x,y,z), il est possible de reconstruire une expression complexe U(x,y,z) de l'onde lumineuse 22 en tout point de coordonnées (x,y,z) de l'espace, et en particulier dans un plan situé à une distance |z| du photodétecteur, et parallèle au plan P selon lequel s'étend le photodétecteur.

L'opérateur de propagation h(x,y,z) a pour fonction de décrire la propagation de la lumière entre le photodétecteur 16 et un point de coordonnées (x,y,z). Il est alors possible de déterminer l'amplitude u(x,y,z) et la phase $\varphi$ (x,y,z) de cette onde lumineuse 22 à cette distance |z|, dite distance de reconstruction, avec :

-

$$u(x,y,z)= abs\ [U(x,y,z)]$$

-

$$\varphi(x,y,z) = arg\ [U(x,y,z)]$$

Les opérateurs *abs* et *arg* désignant respectivement le module et l'argument.

L'application de l'opérateur de propagation permet en particulier de déterminer l'expression complexe U(x,y,z) à une distance |z| du photodétecteur, en amont de ce dernier. On reconstruit alors la valeur complexe de l'onde lumineuse 22 avant que cette dernière n'atteigne le détecteur. On parle alors de rétro-propagation. En attribuant la coordonnée z = 0 au plan de détection P, cette rétro-propagation est mise en œuvre en appliquant un opérateur de propagation h(x,y,-|z|).

Les termes en amont et en aval sont à comprendre selon le sens de propagation de l'onde incidente (12).

Si I(x,y) = I(x,y,z=0) correspond à l'intensité du signal mesuré par le photodétecteur, une relation entre l'intensité mesurée I(x,y) et l'expression complexe de l'onde lumineuse U(x,y), au niveau du plan de détection P est donnée par : $I(x,y) = |U(x,y)|^2$.

L'expression complexe de l'onde lumineuse (22), à une coordonnée (x,y,z) est donnée par

$$U(x, y, z) = \sqrt{I(x, y)} * h(x, y, z),$$ le symbole * désignant un produit de convolution.

avec :

- z < 0 dans le demi-espace délimité par le plan de détection P, comprenant l'échantillon 14,
- z > 0 dans le demi-espace délimité par le plan de détection P et ne comprenant pas l'échantillon 14.

Dans le demi espace délimité par le plan de détection P et comprenant l'échantillon 14, l'expression complexe de l'onde lumineuse peut également s'écrire :

$$U(x,y,z) = \sqrt{I(x,y)} * h(x,y,-|z|)$$

De préférence un pré-traitement mathématique est préalablement appliqué à l'intensité mesurée I(x,y) avant la reconstruction holographique. Cela permet d'améliorer la qualité des résultats, notamment en réduisant les artéfacts lors de l'application de l'opérateur de propagation.

Ainsi, on détermine une intensité $\tilde{I}(x, y)$, dite intensité normalisée, telle que

$$\tilde{I}(x,y) = (I(x,y) - Average\,(I))/Average(I)$$

avec

- I(x,y) = intensité mesurée par le photodétecteur à la coordonnée (x,y),
- Average (I) = moyenne de l'intensité mesurée dans une région d'intérêt de l'image I, incluant ladite coordonnée (x,y). Cette région d'intérêt peut correspondre à l'image entière formée par le photodétecteur.

Ce pré-traitement s'apparente à une normalisation de l'intensité mesurée par l'intensité de l'onde lumineuse incidente 12, cette dernière étant estimée par la grandeur Average (I). Il permet de limiter les artéfacts générés par le processus de reconstruction.

La reconstruction numérique peut notamment reposer sur le modèle de diffraction de Fresnel. Dans cet exemple, l'opérateur de propagation est la fonction de Fresnel-Helmholtz, telle que :

$$h(x,y,z) = \frac{1}{j\lambda z}e^{j2\pi\frac{z}{\lambda}}\exp(j\pi\frac{x^2+y^2}{\lambda z}).$$

où $\lambda$ désigne la longueur d'onde.

Ainsi,

$$U(x,y,z) = \frac{1}{j\lambda z}e^{j2\pi\frac{z}{\lambda}}\iint\sqrt{\tilde{I}(x',y')}\exp(j\pi\frac{(x-x')^2+(y-y'^2)}{\lambda z})dx'dy'$$

où

- x' et y' désignent les coordonnées dans le plan du photodétecteur,
- x et y désignent les coordonnées dans le plan de reconstruction, ce dernier étant situé à une distance |z| du photodétecteur,
- z désigne la coordonnée de l'image reconstruite selon l'axe de propagation Z de l'onde lumineuse incidente 12.

A partir des valeurs de l'expression complexe U(x,y,z), il est possible d'extraire des grandeurs caractéristiques de l'onde lumineuse 22 résultant de la diffraction, par les particules (1,2..9), du de l'onde lumineuse incidente 12 émise par la source 11. Comme précédemment évoqué, on peut évaluer l'amplitude u(x,y,z) ou la phase $\varphi$(x,y,z), mais il est également possible d'évaluer toute fonction de l'amplitude ou de la phase.

On peut, par exemple, évaluer une grandeur caractéristique, dite amplitude complémentaire, $\tilde{u}$(x,y,z) telle que :

$$\tilde{u}(x,y,z) = abs(1 - U(x,y,z))$$

A partir de chaque expression complexe reconstruite U(x,y,z), il est possible de constituer :

- une image $u_z$ de l'amplitude de l'onde 22, selon un plan parallèle au plan du détecteur, à une distance |z| de ce dernier, avec $u_z$(x,y) = abs [U(x,y,z)],

- une image $\varphi_z$ de la phase de l'onde 22, selon un plan parallèle au plan du détecteur, à une distance |z| de ce dernier, avec $\varphi_z$ (x,y) = arg [U(x,y,z)],

- une image $\widetilde{u_z}$ de l'amplitude complémentaire, telle que précédemment définie, de l'onde 22, selon un plan parallèle au plan du détecteur, à une distance |z| de ce dernier, avec $\widetilde{u_z}(x, y, z) = $ abs [1- U(x,y,z)].

Les figures 3A à 3C représentent respectivement des images $\varphi_z$ de la phase reconstruite dans des plans parallèles au photodétecteur matriciel, avec respectivement |z| =3050 $\mu$m, |z| = 3450 $\mu$m (z = d) et |z| = 3850 $\mu$m, sachant que les cellules sont situées dans le plan |z| = 3450 $\mu$m.

On observe, sur chaque image $\varphi_z$ reconstruite, une figure de diffraction élémentaire (31, 32, 33, 34, 35) correspondant à chaque cellule (1,2,3,4,5) de l'échantillon, la partie centrale de chaque figure permettant de déterminer les coordonnées respectives ($x_1$, $y_1$), ($x_2$, $y_2$), ($x_3$, $y_3$), ($x_4$, $y_4$) et ($x_5$, $y_5$) des cellules 1 à 5 dans le plan de détection P. On détermine la valeur de la phase $\varphi(x_1, y_1,z)$, $\varphi(x_2, y_2,z)$, $\varphi(x_3, y_3,z)$, $\varphi(x_4, y_4,z)$, $\varphi(x_5, y_5,z)$ aux différentes valeurs z considérées :

- à |z| = 3850 $\mu$m, la phase associée à chaque cellule est positive, de valeur proche de $\pi$ /5,

- à |z| = 3450 $\mu$m (z = d), la phase associée aux cellules 1, 2 et 3 est négative (voisine de - $\pi$ /5), tandis que la phase associée aux cellules 4 et 5 est positive (voisine de + $\pi$ /5),

- à |z| = 3050 $\mu$m, la phase associée à chaque cellule est négative, de valeur proche de - $\pi$ /5.

Ainsi, sur le plan |z| = 3450 $\mu$m, correspondant au plan dans lequel sont effectivement situées les cellules (z= d), la phase de l'onde lumineuse 22 reconstruite, passant respectivement par les cellules 1, 2 et 3, est négative, tandis que la phase de l'onde lumineuse 22 reconstruite, passant respectivement par les cellules 4 et 5 est négative

Par ailleurs, on a procédé, suite à ces reconstructions, à une coloration des cellules au bleu Trypan, puis à leur observation à l'aide d'un microscope, en utilisant un grossissement x 10. Le bleu de Trypan est un colorant couramment utilisé dans le domaine de la viabilité cellulaire. Les cellules repérées 1, 2 et 3 apparaissent vivantes, tandis que les cellules repérées 4 et 5 présentent une tache sombre, indiquant une mort cellulaire. Ces observations servent de mesure de référence dans les analyses détaillées ci-dessous.

En reconstruisant une image du rayonnement auquel est exposé le détecteur sur le plan contenant les cellules (z = 3450 $\mu$m), et en identifiant, sur cette image reconstruite, la position de chaque cellule, on peut discriminer les cellules vivantes (phase négative) des cellules mortes (phase positive).

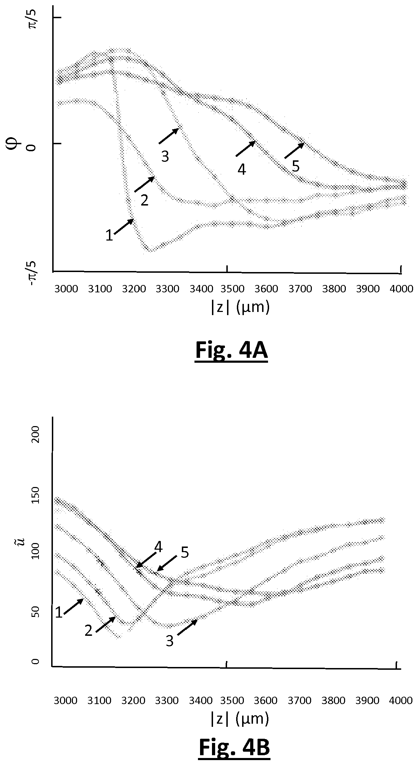

La figure 4A illustre, pour chacune cellule n (1 ≤ n ≤ 5), l'évolution du profil $\varphi(x_n, y_n,z)$ de la phase en fonction de z, pour |z| compris entre 3000 $\mu$m et 4000 $\mu$m. Les profils correspondant aux cellules vivantes (n =1, 2 et 3) se distinguent par une pente marquée à |z| < 3450 $\mu$m, tandis que les profils correspondant aux cellules mortes (n = 4 ou 5) se distinguent par une diminution progressive de la phase selon l'axe de propagation Z, dans le sens allant du photodétecteur matriciel vers l'échantillon.

Ainsi, il est possible d'établir un profil représentant l'évolution de la phase de l'onde 22 à laquelle est exposé le détecteur selon un axe, parallèle à l'axe de propagation Z, passant par chaque cellule. Ce profil peut alors être utilisé pour effectuer une classification entre une cellule vivante et une cellule morte. Ce profil peut notamment être comparé à une bibliothèque de profils réalisés sur des cellules « étalons » dont l'état est connu. Autrement dit, le profil représentant l'évolution de la phase selon l'axe de propagation de l'onde lumineuse constitue une signature de l'état de la cellule.

Le fait de reconstruire une grandeur caractéristique de l'onde 22 résultant de la diffraction d'une particule avec l'onde incidente 12 non pas à une seule distance de reconstruction, mais le long de l'axe de propagation de l'onde incidente, à une pluralité de distances de reconstruction, permet d'obtenir une information plus riche. Cela permet une classification fiable entre les différents états d'une cellule. Par ailleurs, cela évite de connaître précisément la distance séparant une cellule à caractériser du photodétecteur.

Un autre indicateur peut être la distance $|z_0|$ à laquelle la valeur phase $\varphi(x_n, y_n,z)$ passe par zéro, une cellule étant considérée comme vivante si $|z_0|$ est inférieure à la distance d séparant effectivement la cellule du photodétecteur (en l'occurrence d = 3450 $\mu$m), et morte dans le cas contraire.

La figure 4B représente pour chacune cellule n (1 ≤ n ≤ 5), l'évolution du profil $\tilde{u}$ ($x_n$,$y_n$, z) de l'amplitude complémentaire en fonction de z, pour |z| compris entre 3000 $\mu$m et 4000 $\mu$m. Les profils correspondant aux cellules vivantes (n =1, 2 et 3) se distinguent par une valeur minimum $\tilde{u}_{min}$ ≤ 50, tandis que pour les cellules mortes, la valeur minimale $\tilde{u}_{min}$ du profil est supérieure à 50. Autrement dit, il est possible de définir une valeur seuil $\tilde{u}_{threshold}$, la comparaison de cette valeur seuil $\tilde{u}_{threshold}$ avec un point remarquable du profil, en l'occurrence la valeur minimale, permettant de classer la cellule comme vivante ou comme morte.

Sur la figure 4B, on remarque également que lorsque les cellules sont vivantes, la valeur minimale $\tilde{u}_{min}$ du

profil est atteinte à |z| < 3450 $\mu$m, ce qui n'est pas le cas des cellules mortes. Autrement dit, il est possible d'identifier la position $z_{min}$, le long de l'axe de propagation Z, d'un point remarquable du profil, en l'occurrence un minimum, et de comparer cette position à la distance d entre la cellule analysée et le photodétecteur. Si $|z_{min}| \leq d$, la cellule est considérée comme viable. A défaut, elle est considérée comme morte.

Il est donc possible d'établir un profil représentant l'évolution de l'amplitude complémentaire $\tilde{u}$ de l'onde lumineuse 22 à laquelle est exposé le détecteur, selon l'axe de propagation Z et passant par chaque cellule, et d'utiliser ce profil pour effectuer une classification entre une cellule vivante et une cellule morte. Ce profil peut notamment être comparé à une bibliothèque de profils réalisés sur des cellules « étalons » dont l'état est connu. Autrement dit, le profil représentant l'évolution de l'amplitude complémentaire $\tilde{u}$ selon l'axe de propagation constitue une signature de l'état de la cellule.

Dans un deuxième exemple, le dispositif est similaire à celui précédemment mis en œuvre. Les cellules caractérisées sont des cellules de type PC12. De même que dans le premier exemple précédent, on a acquis une image sur le photodétecteur matriciel, selon une configuration identique à celle représentée sur la figure 1. Cette image a permis la reconstruction d'une expression complexe U(x,y,z) de l'onde 22, à laquelle est exposé le photodétecteur, selon l'axe de propagation Z, la distance de reconstruction variant de 3000 $\mu$m à 3800 $\mu$m.

Une mesure de référence a ensuite été réalisée, en utilisant un marquage au bleu de Trypan, permettant d'identifier les cellules mortes D des cellules vivantes A.

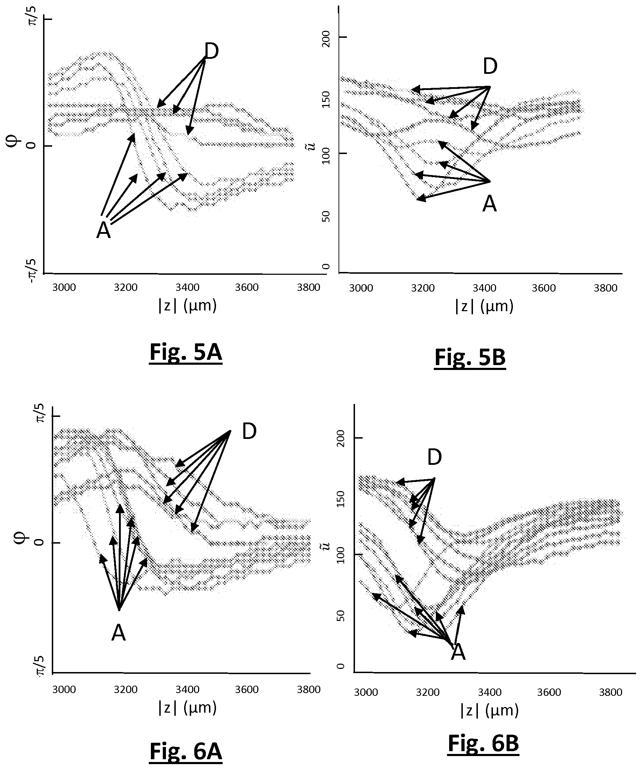

La figure 5A représente l'évolution de la phase $\varphi(x_n, y_n, z)$ en fonction de |z|, $(x_n, y_n)$ désignant les coordonnées du centre de chaque cellule n examinée. De même que dans l'exemple précédent, on observe que :

- la phase $\varphi(x_n, y_n, |z| = d = 3450\ \mu m)$ est négative pour les cellules vivantes, et positive ou nulle pour les cellules mortes ;
- le profil $\varphi(x_n, y_n, z)$ associé à chaque cellule vivante se distingue par une décroissance marquée de la phase à |z| < d, tandis que le profil $\varphi(x_n, y_n, z)$ associé à chaque cellule morte se distingue par une évolution plus lente du profil en fonction de z. Le profil représentant l'évolution de la phase selon l'axe de propagation Z constitue donc une signature de l'état de la cellule ;
- la valeur $|z_0|$ à laquelle la phase de l'onde 22 reconstruite est égale à 0 varie en fonction de l'état des cellules : $|z_0|$ < d pour les cellules vivantes, $|z_0| \geq d$ pour les cellules mortes.

On dispose ainsi de 3 critères de classification d'une cellule: valeur de la phase à |z| = d, évolution de la phase en fonction de z et valeur $|z_0|$ à laquelle la valeur de la phase de l'expression complexe de l'onde 22 reconstruite est nulle.

La figure 5B représente l'évolution de l'amplitude complémentaire $\tilde{u}(x_n, y_n, z)$, telle que précédemment définie, en fonction de z, $(x_n, y_n)$ désignant les coordonnées du centre de chaque cellule n caractérisée. De même que dans l'exemple précédent, l'évolution du profil de l'amplitude complémentaire est différente selon que la cellule soit vivante ou morte. En particulier, lorsque la valeur minimale $\tilde{u}_{min}$ du profil est inférieure à une valeur seuil $\tilde{u}_{threshold}$, ici de l'ordre de 100, une cellule est déclarée vivante, et morte dans le cas contraire.

Dans un troisième exemple, le dispositif est similaire à celui précédemment mis en œuvre. Les cellules caractérisées sont des cellules de type CHO (Chinese Hamster Ovary - lignée cellulaire issue d'ovaires de hamster de Chine). De même que dans les deux exemples précédents, on a acquis une image sur le photodétecteur matriciel, selon une configuration identique à celle représentée sur la figure 1. Cette image a permis la reconstruction d'une expression complexe U(x,y,z) de l'onde 22, à laquelle est exposé le photodétecteur, selon l'axe de propagation Z, la distance de reconstruction |z| variant de 3000 $\mu$m à 3800 $\mu$m.

Une mesure de référence a ensuite été réalisée, en utilisant un marquage au bleu de Trypan, permettant d'identifier les cellules mortes D et les cellules vivantes A.

La figure 6A représente l'évolution de la phase $\varphi(x_n, y_n, z)$ en fonction de z, $(x_n, y_n)$ désignant les coordonnées du centre de chaque cellule n caractérisée. De même que dans l'exemple précédent, on observe que :

- la phase $\varphi(x_n, y_n, |z| = d = 3450\ \mu m)$ est négative pour les cellules vivantes, et positive pour les cellules mortes,
- le profil $\varphi(x_n, y_n, z)$ associé à chaque cellule vivante se distingue par une décroissance marquée de la phase à |z| < d, tandis que le profil $\varphi(x_n, y_n, z)$ associé à chaque cellule morte se distingue par une évolution plus lente du profil en fonction de z. Le profil représentant l'évolution de la phase selon l'axe de propagation Z constitue donc une signature de l'état de la cellule,
- la valeur $|z_0|$ à laquelle la phase de l'onde 22 est égale à 0 varie en fonction de l'état des cellules: $|z_0|$ < d pour les cellules vivantes, $|z_0|$ > d pour les cellules mortes.

La figure 6B représente l'évolution de l'amplitude complémentaire, $\tilde{u}(x_n, y_n, z)$ telle que précédemment définie, en fonction de z, $(x_n, y_n)$ désignant les coordonnées du centre de chaque cellule n caractérisée. De même que dans l'exemple précédent, l'évolution du profil de l'amplitude complémentaire est différente selon que la cellule soit vivante ou morte. En particulier, lorsque la valeur minimale $\tilde{u}_{min}$ du profil est inférieure à une valeur seuil $\tilde{u}_{threhsold}$, ici égal à 30, une cellule est déclarée vivante, ou morte dans le cas contraire.

Selon une variante, la classification entre une cellule vivante et une cellule morte est réalisée en combinant, pour une hauteur z donnée, différents paramètres du rayonnement lumineux 22, auquel le détecteur est exposé. Selon un exemple, on détermine la phase $\varphi(x,y,z)$ ainsi que l'amplitude complémentaire $\tilde{u}(x,y,z)$ le long de l'axe de propagation Z, la classification étant réalisée en mettant en œuvre le ratio entre ces deux paramètres.

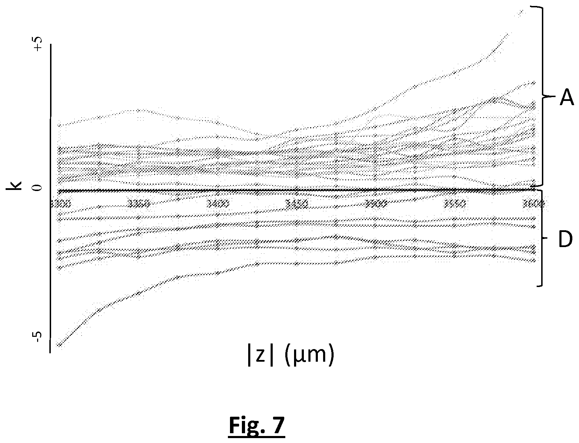

La figure 7 représente le profil, selon l'axe de propagation Z, de la grandeur composite k(x,y,z) telle que

$$k(x, y, z) = \frac{\varphi(x,y,z)}{\tilde{u}(x,y,z)} - k(x_6, y_6, z)$$

Le terme $k(x_6, y_6, z)$ représentant le ratio $\frac{\varphi(x,y,z)}{\tilde{u}(x,y,z)}$ déterminé dans une partie 6 de l'échantillon exempte de cellule. Ce ratio peut être appelé ratio de référence.

Cette figure montre l'évolution de la grandeur composite $k(x_n,y_n,z)$ pour n cellules, chaque cellule n étant identifiée par sa position $(x_n,y_n)$ dans le plan du photodétecteur.

La valeur de la grandeur composite, à une distance de reconstruction z déterminée, est systématiquement supérieure pour les cellules vivantes que pour les cellules mortes. Il est alors possible de définir un seuil $k_{threshold}(z)$, tel que si $k(x_n,y_n, z) \geq k_{threshold}(z)$, la cellule centrée sur la position $(x_n,y_n)$, dans le plan P, est vivante, ou morte dans le cas contraire.

L'application d'un opérateur de propagation numérique h à une image *I* acquise, ou hologramme, par un photodétecteur matriciel 16 peut avoir certaines limites, du fait que l'image acquise ne comporte pas d'information relative à la phase. Aussi, préalablement à l'établissement du profil, il est préférable de disposer d'une information relative à la phase de l'onde lumineuse 22 à laquelle est exposé le photodétecteur 16. Cette information relative à la phase peut être obtenue en reconstruisant une image complexe $U_z$ de l'échantillon 14, selon des méthodes décrites dans l'art antérieur, de façon à obtenir une estimation de l'amplitude et de la phase de l'onde lumineuse 22 au niveau du plan *P* du photodétecteur matriciel 16 ou dans un plan de reconstruction $P_z$ situé à une distance |z| de ce dernier. Les inventeurs ont mis au point un procédé basé sur le calcul d'une image complexe de référence, décrit en lien avec la figure 8A. Ce procédé comprend les étapes suivantes :

- Acquisition d'une image *I* de l'échantillon 14 par le photodétecteur matriciel 16, cette image formant l'hologramme (étape 100).
- Calcul d'une image complexe, dite de référence, $U_{ref}$ de l'échantillon 14 dans un plan de reconstruction $P_z$ ou dans le plan de détection *P*, cette image complexe de référence comportant des informations de phase et d'amplitude de l'onde lumineuse 22 à laquelle est exposé le photodétecteur matriciel 16 ; cette étape est effectuée en appliquant l'opérateur de propagation *h*, précédemment décrit, à l'image acquise *I* (étapes 110 à 170). Cette image complexe est désignée comme étant une image de référence car elle sert de base à la formation du profil sur la base duquel la particule est caractérisée.
- Sélection d'une position radiale (*x,y*) d'une particule dans le plan de détection ou dans un plan parallèle à ce dernier (étape 180), soit en utilisant l'image complexe de référence $U_{ref}$ soit l'image *I* acquise par le photodétecteur 16.
- Application de l'opérateur de propagation *h* à l'image complexe de référence $U_{ref}$ de façon à calculer des images complexes $U_{ref,z}$, dites secondaires, le long de l'axe de propagation Z (étape 185).
- A partir de chaque image complexe secondaire $U_{ref,z}$, estimation d'une grandeur caractéristique de l'onde lumineuse 22, à la position radiale (*x,y*) de la particule préalablement sélectionnée, et à une pluralité de distances du plan de reconstruction $P_z$ (ou du plan de détection *P*), puis formation d'un profil représentant une évolution de ladite grandeur caractéristique selon l'axe de propagation Z (étape 190).
- Caractérisation de la particule en fonction de ce profil. Comme précédemment indiqué, cette caractérisation peut être effectuée par une comparaison du profil obtenu avec des profils étalons obtenus lors d'une phase de calibration, à l'aide d'échantillons étalons. (étape 200).

L'algorithme présenté sur la figure 8A est détaillé ci-dessous, les résultats obtenus au cours de certaines étapes étant illustrés sur les figures 8B à 8F. Les étapes 110 à 170 constituent une façon préférée d'obtenir une image complexe de référence, notée $U_{ref}$ cette image représentant une distribution spatiale de l'expression complexe de l'onde 22 dans un plan de reconstruction $P_z$. L'homme du métier comprendra que d'autres algorithmes permettant de reconstruire une telle image complexe, et par exemple les algorithmes cités en lien avec l'art antérieur, sont également envisageables.

Etape 100 : acquisition d'image

Au cours de cette étape, le capteur d'image 16 acquiert une image *I* de l'échantillon 14, et plus précisément de l'onde lumineuse 22 transmise par ce dernier, à laquelle est exposé le capteur d'image. Une telle image, ou hologramme, est représentée sur la figure 8B.

Cette image a été réalisée en utilisant un échantillon 10 comportant de cellules CHO (cellules ovariennes de hamster) baignant dans un tampon salin, l'échantillon étant contenu dans une chambre fluidique d'épaisseur 100 $\mu$m disposé à une distance d de 1500 $\mu$m d'un capteur CMOS. L'échantillon a été illuminé par une diode électroluminescente 11 dont la bande spectrale d'émission est centrée sur une longueur d'onde de 450 nm et située à une distance D=8 cm de l'échantillon.

Etape 110 : initialisation

Au cours de cette étape, on définit une image initiale $U_0^{k=0}$ de l'échantillon 14, à partir de l'image *I* acquise par le capteur d'image 16. Cette étape est une initialisation de l'algorithme itératif décrit ci-après en lien avec les étapes 120 à 180, l'exposant *k* désignant le rang de chaque itération. Le module $u_0^{k=0}$ de l'image initiale $U_0^{k=0}$ peut-être obtenu en appliquant l'opérateur racine carrée à l'image acquise *I* par le capteur d'image, auquel cas $u_0^{k=0} = \sqrt{I_0}$.

La phase $\varphi_0^{k=0}$ de l'image initiale $U_0^{k=0}$ est soit considérée comme nulle en chaque pixel (*x*,*y*), soit prédéterminée selon une valeur arbitraire. En effet, l'image initiale $U_0^{k=0}$ résulte directement de l'image *I* acquise par le photodétecteur matriciel 16. Or, cette dernière ne comporte pas d'information relative à la phase de l'onde lumineuse 22 transmise par l'échantillon 14, le capteur d'image 16 n'étant sensible qu'à l'intensité de cette onde lumineuse.

Etape 120 : propagation

Au cours de cette étape, l'image $U_0^{k-1}$ obtenue dans le plan de l'échantillon est propagée dans un plan de reconstruction $P_z$, par l'application d'un opérateur de propagation tel que précédemment décrit, de façon à obtenir une image complexe $U_z^k$, représentative de l'échantillon 14, dans le plan de reconstruction $P_z$. La propagation est réalisée par convolution de l'image $U_0^{k-1}$ par l'opérateur de propagation $h_{-z}$, de telle sorte que :

$$U_z^k = U_0^{k-1} * h_{-z},$$

le symbole * désignant un produit de convolution. L'indice -*z* représente le fait que la propagation est réalisée dans un sens opposé à l'axe de propagation Z. On parle de rétro-propagation.

Lors de la première itération (*k* =1), $U_0^{k=0}$ est l'image initiale déterminée lors de l'étape 110. Au cours des itérations suivantes, $U_0^{k-1}$ est l'image complexe dans le plan de détection *P* mise à jour au cours de l'itération précédente.

Le plan de reconstruction $P_z$ est un plan distant du plan de détection *P*, et de préférence parallèle à ce dernier. De préférence, le plan de reconstruction $P_z$ est un plan $P_{14}$ selon lequel s'étend l'échantillon 14. En effet, une image reconstruite dans ce plan permet d'obtenir une résolution spatiale généralement élevée. Il peut également s'agir d'un autre plan, situé une distance non nulle du plan de détection, et de préférence parallèle à ce dernier, par exemple un plan s'étendant entre le photodétecteur matriciel 16 et l'échantillon 14.

La figure 8C représente une le module d'une image $U_z^{k=1}$ reconstruite à une distance de 1440 $\mu$m du plan de détection *P* en appliquant l'opérateur de propagation défini ci-dessus à l'hologramme de la figure 8B. Cette image représente l'image complexe, dans le plan de reconstruction, établie lors de la première itération.

Etape 130 : Calcul d'un indicateur en plusieurs pixels

Au cours de cette étape, on calcule une grandeur $\varepsilon^k(x,y)$ associée à chaque pixels d'une pluralité de pixels

$(x,y)$ de l'image complexe $U_z^k$ , et de préférence en chacun de ces pixels. Cette grandeur dépend de la valeur $U_z^k(x,y)$ de l'image $U_z^k$ , ou de son module, au pixel $(x,y)$ à laquelle elle est calculée. Elle peut également dépendre d'une dérivée dimensionnelle de l'image en ce pixel, par exemple le module d'une dérivée dimensionnelle de cette image.

Dans cet exemple, la grandeur associée à chaque pixel $(x,y)$ est basé sur le module d'une dérivée dimensionnelle, telle que :

$$\varepsilon^k(x,y) = \sqrt{\left|\frac{\partial U_z^k(x,y)}{\partial x}\right|^2 + \left|\frac{\partial U_z^k(x,y)}{\partial y}\right|^2}$$

L'image étant discrétisé en pixels, les opérateurs de dérivée peuvent être remplacés par des opérateurs de Sobel, de telle sorte que :

$$\varepsilon^k(x,y) = \sqrt{(S_x * U_z^k(x,y))(S_x * U_z^k(x,y))^* + (S_y * U_z^k(x,y))(S_y * U_z^k(x,y))^*}$$

où :

- $()^*$ désigne l'opérateur complexe conjugué ;
- $S_x$ et $S_y$ désignent des opérateurs de Sobel selon deux axes orthogonaux X et Y du plan de reconstruction $P_z$.

$$S_x = \begin{bmatrix} 1 & 0 & -1 \\ 2 & 0 & -2 \\ 1 & 0 & -1 \end{bmatrix}$$

Selon cet exemple, et $S_y$ est la matrice transposée de $S_x$.

La figure 8D représente, sous la forme d'une image, la valeur du module $\varepsilon^k(x,y)$ en chaque pixel de l'image $A_z^{k=1}$ représentée sur la figure 8C.

Etape 140 : établissement d'un indicateur de bruit associé à l'image $U_z^k$ .

Lors de l'étape 130, on a calculé des grandeurs $\varepsilon^k(x,y)$ en plusieurs pixels de l'image complexe $U_z^k$ Ces grandeurs peuvent former un vecteur **E**$^k$, dont les termes sont les grandeurs $\varepsilon^k(x,y)$ associées à chaque pixel $(x,y)$. Dans cette étape, on calcule un indicateur, dit indicateur de bruit, à partir d'une norme du vecteur **E**$^k$. D'une façon générale, à une norme est associé un ordre, de telle sorte que la norme $\|\mathbf{x}\|_p$ d'ordre $p$ d'un vecteur **x** de dimension $n$ de coordonnées $(x_1, x_2, ... . x_n)$ est telle que : $\|\mathbf{x}\|_p = (\sum_{i=1}^n |x_i|^p)^{1/p}$, avec $p \geq 0$.

Dans le cas présent, on utilise une norme d'ordre 1, autrement dit $p = 1$. Les inventeurs ont en effet estimé que le recours à une norme d'ordre 1, ou d'ordre inférieur ou égal à 1, est particulièrement adapté à un tel échantillon, comme expliqué ci-après.

Au cours de cette étape, la grandeur $\varepsilon^k(x,y)$ calculée à partir de l'image complexe $U_z^k$ à chaque pixel $(x, y)$ de cette dernière, est sommée de façon à constituer un indicateur de bruit $\varepsilon^k$ associé à l'image complexe $U_z^k$.

Ainsi,

$$\varepsilon^k = \sum_{(x,y)} \varepsilon^k(x,y)$$

Cet indicateur de bruit $\varepsilon^k$ correspond à une norme de variation totale de l'image complexe $A_z^k$

En se rapportant à l'exemple de la figure 8D, l'indicateur de bruit $\varepsilon^{k=1}$ est obtenu, lors de la première itération, par une sommation de la valeur des pixels de cette image.

Du fait de l'utilisation d'une norme d'ordre 1, ou d'ordre inférieur ou égal à 1, la valeur de l'indicateur de bruit $\varepsilon^k$ diminue lorsque l'image complexe $U_z^k$ est de plus en plus représentative de l'échantillon. En effet, lors des premières itérations, la valeur de la phase $\varphi_0^k(x,y)$, en chaque pixel (x,y) de l'image $U_0^k$ est mal estimée. La propagation de l'image de l'échantillon du plan de détection P vers le plan de reconstruction $P_z$ s'accompagne alors d'un bruit de reconstruction important, comme évoqué en lien avec l'art antérieur. Ce bruit de reconstruction se présente sous la forme de fluctuations apparaissant sur l'image reconstruite. Du fait de ces fluctuations, un indicateur de bruit $\varepsilon^k$, tel que précédemment défini est d'autant plus élevé que la contribution du bruit de reconstruction, sur l'image reconstruite, est importante. En effet, les fluctuations dues au bruit de reconstruction tendent à augmenter la valeur de cet indicateur.

Un aspect important de cette étape consiste à déterminer, dans le plan de détection P, des valeurs de phase $\varphi_0^k(x,y)$ de chaque pixel de l'image de l'échantillon $U_0^k$, permettant d'obtenir, lors d'une itération suivante, une image reconstruite $U_z^{k+1}$ dont l'indicateur $\varepsilon^{k+1}$ est inférieur à l'indicateur $\varepsilon^k$.

Lors de la première itération, comme précédemment expliqué, on ne dispose que d'une information pertinente sur l'intensité de l'onde lumineuse 22, mais non sur sa phase. La première image reconstruite $U_z^{k=1}$ dans le plan de reconstruction $P_z$ est donc affectée d'un bruit de reconstruction important, du fait de l'absence d'information pertinente quant à la phase de l'onde lumineuse 22 dans le plan de détection P. Par conséquent, l'indicateur $\varepsilon^{k=1}$ est élevé. Au cours des itérations suivantes, l'algorithme procède à un ajustement progressif de la phase $\varphi_0^k(x,y)$ dans le plan de détection P, de façon à minimiser progressivement l'indicateur $\varepsilon^k$.

L'image $U_0^k$ dans le plan de détection est représentative de l'onde lumineuse 22 dans le plan de détection P, aussi bien du point de vue de son intensité que de sa phase. Les étapes 120 à 160 visent à établir, de façon itérative, la valeur de la phase $\varphi_0^k(x,y)$ de chaque pixel de l'image $U_0^k$, minimisant l'indicateur $\varepsilon^k$, ce dernier étant obtenu sur l'image $U_z^k$ obtenue par propagation de l'image $U_0^{k-1}$ dans le plan de reconstruction $P_z$.

L'algorithme de minimisation peut être un algorithme de descente de gradient, ou de descente de gradient conjugué, ce dernier étant décrit ci-après.

Etape 150 : Ajustement de la valeur de la phase dans le plan de détection.

L'étape 150 vise à déterminer une valeur de la phase $\varphi_0^k(x,y)$ de chaque pixel de l'image complexe $U_0^k$ de façon à minimiser l'indicateur $\varepsilon^{k+1}$ résultant d'une propagation de l'image complexe $U_0^k$ dans le plan de reconstruction $P_z$, au cours de l'itération suivante k+1. Pour cela, un vecteur de phase $\boldsymbol{\varphi_0^k}$ est établi, dont chaque terme est la phase $\varphi_0^k(x,y)$ d'un pixel (x,y) de l'image complexe $U_0^k$. La dimension de ce vecteur est ($N_{pix}$, 1), où $N_{pix}$ désigne le nombre de pixels considérés. Ce vecteur est mis à jour au cours de chaque itération, par l'expression de mise à jour suivante :

$$\varphi_0^k(x,y) = \varphi_0^{k-1}(x,y) + \alpha^k p^k(x,y)$$

où :

- $\alpha^k$ est un entier, désigné par le terme « pas », et représentant une distance ;
- $p^k$ est un vecteur de direction, de dimension ($N_{pix}$, 1), dont chaque terme $p(x, y)$ forme une direction du gradient $\nabla\varepsilon^k$ de l'indicateur $\varepsilon^k$.

Cette équation peut être exprimée sous forme vectorielle, comme suit :

$$\boldsymbol{\varphi_0^k} = \boldsymbol{\varphi_0^{k-1}} + \alpha^k \boldsymbol{p^k}$$

On peut montrer que :

$$p^k = -\nabla\varepsilon^k + \beta^k p^{k-1}$$

où :

- $\nabla\varepsilon^k$ est un vecteur de gradient, de dimension ($N_{pix}$, 1), dont chaque terme représente une variation de l'indicateur $\varepsilon^k$ en fonction de chacun des degrés de liberté des inconnues du problème, c'est-à-dire les termes du vecteur $\varphi_0^k \cdot$ ;
- $p^{k-1}$ est un vecteur de direction établi lors de l'itération précédente ;
- $\beta^k$ est facteur d'échelle appliqué au vecteur de direction $p^{k-1}$.
  Chaque terme $\nabla\varepsilon^k(x,y)$ du vecteur de gradient $\nabla\varepsilon$, est tel que

$$\nabla\varepsilon^k(r') = \frac{\partial\varepsilon^k}{\partial\varphi_0^k(r')} = -\mathrm{Im}\left(U_0^{k^*}(r')\left(\left(S_x * \frac{S_x * U_z^k}{\varepsilon^k} + S_y * \frac{S_y * U_z^k}{\varepsilon^k}\cdot\right) * h_z\right)(r')\right)$$

où Im représente l'opérateur partie imaginaire et $r'$ représente une coordonnée ($x$, $y$) dans le plan de de détection.

Le facteur d'échelle $\beta^k$ peut être exprimé de telle sorte que :

$$\beta^{(k)} = \frac{\nabla\varepsilon^{(k)}.\nabla\varepsilon^{(k)}}{\nabla\varepsilon^{(k-1)}.\nabla\varepsilon^{(k-1)}}$$

Le pas $\alpha^k$ peut varier selon les itérations, par exemple entre 0.03 au cours des premières itérations et 0.0005 lors des dernières itérations.

L'équation de mise à jour permet d'obtenir un ajustement du vecteur $\varphi_0^k$, ce qui entraîne une mise à jour itérative de la phase $\varphi_0^k(x,y)$ en chaque pixel de l'image complexe $U_0^k\cdot$ Cette image complexe $U_0^k$, dans le plan de détection, est alors mise à jour par ces nouvelles valeurs de la phase associée à chaque pixel. Notons que le module de l'image complexe $U_0^k$ n'est pas modifié, ce dernier étant déterminé à partir de l'image acquise par le photodétecteur matriciel 16, de telle sorte que $u_0^k(x,y) = u_0^{k=0}(x,y)$.

Etape 160 : Réitération ou sortie d'algorithme.

Tant qu'un critère de convergence n'est pas atteint, l'étape 160 consiste à réitérer l'algorithme, par une nouvelle itération des étapes 120 à 160, sur la base de l'image complexe $U_0^k$ mise à jour lors de l'étape 150. Le critère de convergence peut être un nombre K prédéterminé d'itérations, ou une valeur minimale du gradient $\nabla\varepsilon^k$ de l'indicateur, ou une différence considérée comme négligeable entre deux vecteurs de phase $\varphi_0^{k-1}$, $\varphi_0^k$ consécutifs. Lorsque le critère de convergence est atteint, on dispose d'une estimation considérée comme correcte d'une image complexe de l'échantillon, dans le plan de détection $P$ ou dans le plan de reconstruction $P_z$.

Etape 170 : Obtention de l'image complexe de référence.

A l'issue de la dernière itération, le procédé peut comprendre une propagation de l'image complexe $U_0^k$ résultant de la dernière itération dans le plan de reconstruction $P_z$, de manière à obtenir une image complexe de référence $U_{ref} = U_z^k\cdot$ $U_{ref}$ De façon alternative, l'image complexe de référence $U_{ref}$ est l'image complexe $U_0^k$ résultant de la dernière itération dans le plan de détection $P$. Lorsque la densité des particules est élevée, cette alternative est cependant moins avantageuse car la résolution spatiale dans le plan de détection $P$ est moins élevée que dans le plan de reconstruction $P_z$, notamment lorsque le plan de reconstruction $P_z$ correspond à un plan $P_{14}$ selon lequel s'étend l'échantillon 14.

La figure 8E représente une image du module $u_Z^{k=30}$ de chaque pixel de l'image complexe de référence $U_Z^{k=30}$ obtenue dans un plan de reconstruction $P_z$ à l'issue de 30 itérations. Cette image peut être comparée à la figure 8C, montrant une image similaire $A_Z^{k=1}$ obtenue lors de la première itération. On observe une nette diminution du bruit de reconstruction, en particulier entre chaque particule. Par ailleurs, la résolution spatiale de cette image permet une bonne identification des coordonnées radiales ($x,y$) de chaque particule.

Etape 180 : Sélection des coordonnées radiales particule.

Au cours de cette étape, on sélectionne les coordonnées radiales ($x,y$) d'une particule à partir de l'image de référence $U_{ref} = U_Z^{k=30}$, par exemple à partir de l'image de son module $u_{ref} = u_Z^{k=30}$ ou de l'image de sa phase $\varphi_{ref} = \varphi_Z^{k=30}$. Comme précédemment évoqué, le terme coordonnée radiale désigne une coordonnée dans le plan de détection ou dans le plan de reconstruction. Il est également envisageable d'effectuer cette sélection à partir de l'holo-gramme $I_0$ ou à partir de l'image complexe $U_0^k$ obtenue dans le plan de détection suite à la dernière itération. Cependant, lorsque le nombre de particules augmente, il est préférable d'effectuer cette sélection sur l'image formée dans le plan de reconstruction, du fait de sa meilleure résolution spatiale, en particulier lorsque le plan de reconstruction correspond $P_z$ au plan de l'échantillon $P_{14}$. Sur la figure 8E, on a représenté la sélection d'une particule, entourée par un contour en pointillés.

Etape 185 : Application d'un opérateur de propagation

Au cours de cette étape 185, l'image complexe de référence $U_{ref}$ est propagée selon une pluralité de distances de reconstruction, en utilisant un opérateur de propagation $h$ tel que précédemment défini, de façon à disposer d'une pluralité d'images complexes, dites secondaires, $U_{ref,z}$ reconstruites à différentes distances du plan de détection $P$ ou du plan de reconstruction $P_z$. Ainsi, cette étape comprend la détermination d'une pluralité d'images complexes $U_{ref,z}$ telles que :

$$U_{ref,z} = U_{ref} * h_z \text{ avec } z_{min} \leq z \leq z_{max}.$$

Les valeurs $z_{min}$ et $z_{max}$ sont les coordonnées minimales et maximales, selon l'axe Z, selon lesquelles l'image complexe de référence est propagée. De préférence, les images complexes sont reconstruites selon une pluralité de coordonnées $z$ entre l'échantillon 14 et le capteur d'image 16. Les images complexes peuvent être formées de part et d'autre de l'échantillon 14.

Ces images complexes secondaires sont établies par application d'un opérateur de reconstruction holographique $h$ à l'image de référence $U_{ref}$. Or, cette dernière est une image complexe décrivant correctement l'onde lumineuse 22 auquel est exposé le capteur d'image, en particulier au niveau de sa phase, suite aux itérations des étapes 120 à 160. Par conséquent, les images secondaires $U_{ref,z}$ forment un bon descripteur de la propagation de l'onde lumineuse 22 selon l'axe de propagation Z.

Etape 190 : Formation d'un profil

Au cours de cette étape, à partir de chaque image complexe secondaire $U_{ref,z}$, on détermine une grandeur caractéristique, telle que précédemment définie, de l'onde lumineuse 22 de façon à déterminer un profil représentant l'évolution de ladite grandeur caractéristique selon l'axe de propagation Z. La grandeur caractéristique peut être, par exemple le module ou la phase, ou leur combinaison. La figure 8F représente l'évolution de la phase $\varphi(z)$ de l'onde lumineuse 22 le long de l'axe de propagation Z.

Etape 200 : Caractérisation

La particule peut ensuite être caractérisée à partir du profil formé lors de l'étape précédente. De préférence, on dispose d'une base de données de profils étalons formés au cours d'une phase d'apprentissage à l'aide d'échantillons étalons connus. La caractérisation est alors effectuée par une comparaison ou d'une classification du profil formé sur la base des profils étalons.

Ce mode de réalisation, basé sur une formation d'une image complexe de référence, a été mis en œuvre, en utilisant la norme de variation totale sur des cellules de type CHO, acronyme de cellules ovariennes de hamster, baignant dans un milieu de culture CD CHO (Thermofischer). L'échantillon a été placé dans une chambre fluidique d'épaisseur 100 $\mu$m, disposé à une distance de 8 cm d'une diode électroluminescente, dont la bande spectrale est centrée sur 450 nm. L'échantillon est placé à une distance de 1500 $\mu$m d'un capteur d'image CMOS de 2748x3840 pixels .L'ouverture du filtre spatial 18 a un diamètre de 150 $\mu$m.

La figure 9A représente l'image *I* acquise par le photodétecteur matriciel 16. Les images du module et de la phase de l'image complexe de référence $U_z^k$ reconstruite, dans le plan de l'échantillon $P_{14}$, sont respectivement représentées sur les figures 5B et 5C. Ces images ont été obtenues en 100 itérations. L'homogénéité des niveaux de gris entre chaque cellule atteste de la qualité de la reconstruction. On a appliqué, sur cette image de référence, un opérateur de propagation *h* tel que précédemment décrit de façon à disposer d'une pluralité d'images complexes secondaires $U_{ref,z}$ selon l'axe de propagation Z. Par ailleurs, sur l'image du module ou sur l'image de la phase de l'image de référence, on a identifié deux cellules, respectivement entourées par un contour en tirets noirs (cellule 10b-1) et en pointillés noirs (cellule 10b-2) sur les figures 9B et 9C. Les coordonnées radiales (*x,y*) de ces deux cellules ont été extraites. On a formé, pour chaque cellule, un profil *u(z)* représentatif du module et un profil $\varphi(z)$ représentatif de la phase de l'onde lumineuse 22 atteignant le capteur d'image 16. La valeur de chaque point du profil est respectivement obtenu en déterminant le module et la phase d'une image complexe secondaire $U_{ref,z}$ auxdites coordonnées radiales (*x,y*).

Les figures 9D et 9E représentent respectivement le profil du module et de la phase de la cellule 10b-1. Les figures 9F et 9G représentent respectivement le profil du module et de la phase de la cellule 10b-2.

Par ailleurs, on a procédé, suite à ces reconstructions, à une coloration des cellules au bleu Trypan, puis à leur observation à l'aide d'un microscope, en utilisant un grossissement x 10. L'image obtenue est montrée sur la figure 9H. La cellule 10b-1 est une cellule vivante, tandis que la cellule 10b-2 apparaît comme étant une cellule morte.

Les profils de module ou de phase des figures 9D et 9E peuvent être considérés comme représentatifs d'une cellule CHO vivante, tandis que les figures 9F et 9G peuvent être considérés comme représentatifs d'une cellule CHO morte. La caractérisation de cellules CHO peut être réalisée sur la base de tels profils.

Les exemples décrits ci avant exposent des critères d'identification simples, basés sur l'évolution du profil d'une grandeur caractéristique en fonction de la distance de reconstruction, et de comparaisons à l'aide de seuils pré-établis. Naturellement, d'autres méthodes de classification, plus complexes, peuvent être mises en œuvre, sans sortir du cadre de l'invention définie par les revendications annexées.

## Revendications

1.  Procédé pour déterminer l'état d'une cellule (1,2,3,4,5), ladite cellule étant placée dans un échantillon (14), le procédé comportant les étapes suivantes :

    - illumination dudit échantillon à l'aide d'une source de lumière (11), la source de lumière produisant une onde lumineuse incidente (12) se propageant vers l'échantillon (14) selon un axe de propagation (Z) ;
    - acquisition, à l'aide d'un photodétecteur matriciel (16) d'une image (I) de l'échantillon, l'échantillon étant disposé entre ladite source de lumière et ledit photodétecteur matriciel, de telle sorte que le photodétecteur matriciel est exposé à une onde lumineuse (22) comprenant des interférences entre l'onde lumineuse incidente (12) et une onde de diffraction produite par chaque cellule (1,2,3,4,5) ;
    - identification d'une position $(x_n,y_n)$ de ladite cellule dans un plan parallèle à un plan de détection (P) selon lequel s'étend le photodétecteur matriciel ;

    le procédé étant **caractérisé en ce qu'**il comprend les étapes suivantes :

    - application d'un algorithme de reconstruction numérique à ladite image acquise (I), de façon à déterminer au moins une grandeur caractéristique (u, $\varphi$, $\tilde{u}$, k) de ladite onde lumineuse (22) à laquelle est exposé le photodétecteur matriciel, à ladite position $(x_n,y_n)$, à une pluralité de distances (|z|) de ce dernier, dites distances de reconstruction, selon ledit axe de propagation (Z) ;
    - classification de la cellule en fonction d'un profil représentant une évolution de ladite grandeur caractéristique (u, $\varphi$, $\tilde{u}$, k) en fonction desdites distances, selon l'axe de propagation, la classification permettant de déterminer l'état de la cellule parmi des états prédéterminés.

**2.** Procédé selon la revendication 1, dans lequel les états prédéterminés comprennent un état de vie cellulaire et un état de mort cellulaire.

**3.** Procédé selon l'une quelconque des revendications précédentes, dans lequel la grandeur caractéristique est obtenue en estimant, à ladite distance de reconstruction, une expression complexe (U(x,y,z)) de l'onde lumineuse (22) à laquelle est exposé le photodétecteur matriciel.

**4.** Procédé selon la revendication 3, dans lequel ladite grandeur caractéristique est déterminée à partir du module ou de l'argument de ladite expression complexe (U(x,y,z)).

**5.** Procédé selon l'une quelconque des revendications précédentes, dans lequel la classification est réalisée en comparant ladite évolution de ladite grandeur caractéristique à des profils types prédéterminés.

**6.** Procédé selon l'une quelconque des revendications précédentes, comprenant également une étape de reconstruction d'une image de ladite grandeur caractéristique selon un plan parallèle au plan de détection (P), et à ladite distance de reconstruction, la valeur de ladite grandeur caractéristique à ladite position $(x_n,y_n)$ de la cellule à ladite distance de reconstruction, étant déterminée en fonction de cette image.

**7.** Procédé selon la revendication 6, la position $(x_n,y_n)$ de chaque cellule, dans un plan parallèle au plan de détection (P), étant déterminée à l'aide de l'image ainsi reconstruite.

**8.** Procédé selon l'une quelconque des revendications 1 à 7, comportant :

- la détermination d'une image complexe de référence ($U_{ref}$), dans un plan de reconstruction ou dans le plan de détection, par application d'un algorithme de reconstruction numérique à l'image acquise par le photodétecteur matriciel (16) ;
- à partir de ladite image complexe de référence, l'estimation d'au moins une grandeur caractéristique (u, $\tilde{u}$, φ, k) de l'onde lumineuse (22) à laquelle est exposé le photodétecteur matriciel (16), à une pluralité de distances (|z|) de reconstruction de ce dernier.

**9.** Procédé selon la revendication 8, comportant :

- l'application d'un opérateur de propagation (*h*) à l'image complexe de référence ($U_{ref}$), de façon à calculer des images complexes dites secondaires ($U_{ref,z}$), selon une pluralité de distances du plan de reconstruction ou du plan selon lequel s'étend le photodétecteur matriciel ;
- la détermination d'une grandeur caractéristique à chacune desdites distances, à partir de chaque image complexe secondaire ($U_{ref,z}$).

**10.** Procédé selon l'une quelconque des revendications précédentes, la source de lumière (11) étant une source spatialement cohérente.

**11.** Procédé selon l'une quelconque des revendications précédentes, dans lequel la source de lumière (11) est une diode électroluminescente.

**12.** Procédé selon l'une quelconque des revendications précédentes, dans lequel aucune optique de grossissement n'est disposée entre l'échantillon (14) et le photodétecteur matriciel (16).

**13.** Dispositif pour déterminer l'état d'une cellule, ladite cellule étant disposée dans un échantillon, le dispositif comprenant :

- une source de lumière (11) agencée pour produire une onde lumineuse incidente (12) selon un axe de propagation (Z), en direction dudit échantillon (14) ;
- un photodétecteur matriciel (16), agencé pour acquérir une image de l'échantillon, en étant exposé à une onde lumineuse (22), résultant de l'interférence entre ladite onde lumineuse incidente (12) et une onde de diffraction formée par ladite cellule ;
- un support, pour maintenir l'échantillon entre ladite source de lumière (11) et le photodétecteur matriciel (16) ;
- un processeur (20) configuré pour mettre en œuvre l'étape suivante :

- identification d'une position $(x_n,y_n)$ de ladite cellule dans un plan parallèle à un plan selon lequel s'étend le photodétecteur ;

le dispositif étant **caractérisé en ce que** ledit processeur (20) est configurée pour mettre en œuvre les étapes suivantes :

- application d'un algorithme de reconstruction numérique à ladite image acquise, de façon à déterminer au moins une grandeur caractéristique $(u, \varphi, \tilde{u}, k)$ de l'onde lumineuse (22) à laquelle est exposé le photodétecteur, à ladite position $((x_n,y_n)$, à une pluralité de distances $(|z|)$ de ce dernier, dites distances de reconstruction, selon l'axe de propagation (Z) ;
- classification de ladite cellule en fonction d'un profil représentant l'évolution de la grandeur caractéristique, selon l'axe de propagation, la classification étant apte à déterminer l'état de la cellule parmi des états prédéterminés.

**14.** Dispositif selon la revendication 13, dans lequel le dispositif ne comporte pas d'optique de grossissement entre le photodétecteur matriciel (16) et l'échantillon analysé (14).

**15.** Incubateur, destiné à la croissance de cellules, l'incubateur comprenant au moins un dispositif selon les revendications 13 à 14.

**Patentansprüche**

**1.** Verfahren zum Bestimmen des Zustands einer Zelle (1, 2, 3, 4, 5), wobei die Zelle in einer Probe (14) platziert ist, wobei das Verfahren die folgenden Schritte umfasst:

- Beleuchtung der Probe mit einer Lichtquelle (11), wobei die Lichtquelle eine einfallende Lichtwelle (12) erzeugt, die sich entlang einer Ausbreitungsachse (Z) in Richtung der Probe (14) ausbreitet;
- Erfassung eines Bildes (I) der Probe mittels eines Matrix-Photodetektors (16), wobei die Probe zwischen der Lichtquelle und dem Matrix-Photodetektor angeordnet ist, so dass der Matrix-Photodetektor einer Lichtwelle (22) ausgesetzt ist, die Interferenzen zwischen der einfallenden Lichtwelle (12) und einer von jeder Zelle (1, 2, 3, 4, 5) erzeugten Beugungswelle umfasst;
- Identifizierung einer Position $(x_n, y_n)$ der Zelle in einer Ebene parallel zu einer Erfassungsebene (P), entlang der sich der Matrix-Photodetektor erstreckt;

wobei das Verfahren **dadurch gekennzeichnet ist, dass** es die folgenden Schritte umfasst:

- Anwendung eines digitalen Rekonstruktionsalgorithmus auf das erfasste Bild (I), um mindestens eine charakteristische Größe $(u, \varphi, \hat{u}, k)$ der Lichtwelle (22) zu bestimmen, der der Matrix-Photodetektor an der Position $(x_n, y_n)$ entlang der Ausbreitungsachse (Z) in einer Vielzahl von Abständen $(|z|)$ zu dieser, den Rekonstruktionsabständen, ausgesetzt ist;
- Klassifizierung der Zelle gemäß einem Profil, das eine Entwicklung der charakteristischen Größe $(u, \varphi, \hat{u}, k)$ in Abhängigkeit der Abstände entlang der Ausbreitungsachse darstellt, wobei die Klassifikation es ermöglicht, den Zustand der Zelle aus vorbestimmten Zuständen zu bestimmen.

**2.** Verfahren nach Anspruch 1, wobei die vorbestimmten Zustände einen Zustand des Zelllebens und einen Zustand des Zelltods beinhalten.

**3.** Verfahren nach einem der vorhergehenden Ansprüche, wobei die charakteristische Größe im Rekonstruktionsabstand durch Schätzen eines komplexen Ausdrucks $(U(x,y,z))$ der Lichtwelle (22) erhalten wird, der der Matrix-Photodetektor ausgesetzt ist.

**4.** Verfahren nach Anspruch 3, wobei die charakteristische Größe aus dem Modul oder Argument des komplexen Ausdrucks $(U(x,y,z))$ bestimmt wird.

**5.** Verfahren nach einem der vorhergehenden Ansprüche, bei dem die Klassifizierung durch Vergleichen der Entwicklung der charakteristischen Größe mit vorbestimmten Standardprofilen durchgeführt wird.

6. Verfahren nach einem der vorhergehenden Ansprüche, das auch einen Schritt der Rekonstruktion eines Bildes der charakteristischen Größe entlang einer Ebene parallel zur Erfassungsebene (P) und im Rekonstruktionsabstand umfasst, wobei der Wert der charakteristischen Größe an der Position $(x_n, y_n)$ der Zelle im Rekonstruktionsabstand gemäß dem Bild bestimmt wird.

7. Verfahren nach Anspruch 6, wobei die Position $(x_n, y_n)$ jeder Zelle in einer Ebene parallel zur Erfassungsebene (P) unter Verwendung des so rekonstruierten Bildes bestimmt wird.

8. Verfahren nach einem der Ansprüche 1 bis 7, umfassend:

- Bestimmung eines komplexen Referenzbildes $(U_{ref})$ in einer Rekonstruktions- oder Erfassungsebene durch Anwendung eines digitalen Rekonstruktionsalgorithmus auf das vom Matrix-Photodetektor (16) erfasste Bild;
- aus dem komplexen Referenzbild Schätzung mindestens einer charakteristischen Größe ($u$, $\tilde{u}$, $\varphi$, $k$) der Lichtwelle (22), der der Matrix-Photodetektor (16) ausgesetzt ist in einer Vielzahl von Rekonstruktionsabständen $(|z|)$ desselben.

9. Verfahren nach Anspruch 8, umfassend:

- Anwendung eines Ausbreitungsoperators ($h$) auf das komplexe Referenzbild $(U_{ref})$, um sekundäre komplexe Bilder $(U_{ref,z})$ zu berechnen, entlang einer Vielzahl von Abständen von der Rekonstruktionsebene oder der Ebene, entlang der sich der Matrix-Photodetektor erstreckt;
- Bestimmung einer charakteristischen Größe in jedem der Abstände aus jedem sekundären komplexen Bild $(U_{ref,z})$.

10. Verfahren nach einem der vorhergehenden Ansprüche, wobei die Lichtquelle (11) eine räumlich kohärente Quelle ist.

11. Verfahren nach einem der vorhergehenden Ansprüche, wobei die Lichtquelle (11) eine lichtemittierende Diode ist.

12. Verfahren nach einem der vorhergehenden Ansprüche, bei dem kein optisches Vergrößerungssystem zwischen der Probe (14) und dem Matrix-Photodetektor (16) angeordnet ist.

13. Vorrichtung zum Bestimmen des Zustands einer Zelle, wobei die Zelle in einer Probe angeordnet ist, wobei die Vorrichtung umfasst:

- eine Lichtquelle (11), die dazu angeordnet ist, eine einfallende Lichtwelle (12) entlang einer Ausbreitungsachse (Z) in Richtung der Probe (14) zu erzeugen;
- einen Matrix-Photodetektor (16), der dazu angeordnet ist, ein Bild der Probe zu erfassen, indem er einer Lichtwelle (22) ausgesetzt wird, die sich aus der Interferenz zwischen der einfallenden Lichtwelle (12) und einer von der Zelle gebildeten Beugungswelle ergibt;
- einen Träger, um die Probe zwischen der Lichtquelle (11) und dem Matrix-Photodetektor (16) zu halten;
- einen Prozessor (20), der konfiguriert ist, um den folgenden Schritt auszuführen:

- Identifizierung einer Position $(x_n, y_n)$ der Zelle in einer Ebene parallel zu einer Ebene, entlang der sich der Photodetektor erstreckt; wobei die Vorrichtung **dadurch gekennzeichnet ist, dass** der Prozessor (20) konfiguriert ist, um die folgenden Schritte auszuführen:

- Anwendung eines digitalen Rekonstruktionsalgorithmus auf das erfasste Bild, um mindestens eine charakteristische Größe ($u$, $\varphi$, $\hat{u}$, $k$) der Lichtwelle (22) zu bestimmen, der der Photodetektor an der Position $(x_n, y_n)$ entlang der Ausbreitungsachse (Z) in einer Vielzahl von Abständen $(|z|)$ zu dieser, den Rekonstruktionsabständen, ausgesetzt ist;
- Klassifizierung der Zelle gemäß einem Profil, das eine Entwicklung der charakteristischen Größe entlang der Ausbreitungsachse darstellt, wobei die Klassifizierung dazu geeignet ist, den Zustand der Zelle aus vorbestimmten Zuständen zu bestimmen.

14. Vorrichtung nach Anspruch 13, wobei die Vorrichtung kein optisches Vergrößerungssystem zwischen dem Matrix-Photodetektor (16) und der analysierten Probe (14) umfasst.

15. Inkubator für das Zellwachstum, wobei der Inkubator mindestens eine Vorrichtung nach den Ansprüchen 13 bis 14 umfasst.

**Claims**

1. Method for determining the state of a cell (1,2,3,4,5), said cell being placed in a sample (14), the method including the following steps:

   - illuminating said sample using a light source (11), the light source producing an incident light wave (12) propagating towards the sample (14) along a propagation axis (Z);
   - acquiring, using a matrix-array photodetector (16), an image (I) of the sample, the sample being placed between said light source and said matrix-array photodetector in such a way that the matrix-array photodetector is exposed to a light wave (22) comprising interference between the incident light wave (12) and a diffraction wave produced by each cell (1,2,3,4,5);
   - identifying a position $(x_n, y_n)$ of said cell in a plane parallel to a detection plane (P) in which the matrix-array photodetector lies;

   the method being **characterized in that** it includes the following steps:

   - applying a digital reconstruction algorithm to said acquired image (I), so as to determine at least one characteristic quantity (u, φ, $\tilde{u}$, k) of said light wave (22) to which the matrix-array photodetector is exposed, at said position $(x_n, y_n)$, at a plurality of what are called reconstruction distances (|z|) from said photodetector along said propagation axis (Z); and
   - classifying the cell depending on a profile representing a variation in said characteristic quantity (u, φ, $\tilde{u}$, k) as a function of said distances along the propagation axis, this classification allowing the state of the cell to be determined from among preset states.

2. Method according to Claim 1, wherein the preset states comprise a living cell state and a dead cell state.

3. Method according to either one of the preceding claims, wherein the characteristic quantity is obtained by estimating, at said reconstruction distance, a complex expression (U(x,y,z)) of the light wave (22) to which the matrix-array photodetector is exposed.

4. Method according to Claim 3, wherein said characteristic quantity is determined from the modulus or the argument of said complex expression (U(x,y,z)).

5. Method according to any one of the preceding claims, wherein the classification is carried out by comparing said variation in said characteristic quantity to preset profiles.

6. Method according to any one of the preceding claims, also comprising a step of reconstructing an image of said characteristic quantity in a plane parallel to the detection plane (P), and at said reconstruction distance, the value of said characteristic quantity at said position $(x_n, y_n)$ of the cell, at said reconstruction distance, being determined depending on this image.

7. Method according to Claim 6, the position $(x_n, y_n)$ of each cell, in a plane parallel to the detection plane (P), being determined using the image thus reconstructed.

8. Method according to any one of Claims 1 to 7, including:

   - determining a reference complex image ($U_{ref}$), in a reconstruction plane or in the detection plane, by applying a digital reconstruction algorithm to the image acquired by the matrix-array photodetector (16); and
   - on the basis of said reference complex image, estimating at least one characteristic quantity (u, $\tilde{u}$, φ, k) of the light wave (22) to which the matrix-array photodetector (16) is exposed, at a plurality of reconstruction distances (|z|) from the latter.

9. Method according to Claim 8, including:

   - applying a propagation operator (h) to the reference complex image ($U_{ref}$), so as to calculate what are called secondary complex images ($U_{ref,z}$) for a plurality of distances from the reconstruction plane or from the plane in which the matrix-array photodetector lies; and
- determining a characteristic quantity at each of said distances, from each secondary complex image ($U_{ref,z}$).

10. Method according to any one of the preceding claims, the light source (11) being a spatially coherent source.

11. Method according to any one of the preceding claims, wherein the light source (11) is a light-emitting diode.

12. Method according to any one of the preceding claims, wherein no magnifying optics are placed between the sample (14) and the matrix-array photodetector (16).

13. Device for determining the state of a cell, said cell being placed in a sample, the device comprising:

- a light source (11) that is arranged to produce an incident light wave (12), along a propagation axis (Z), in the direction of said sample (14);
- a matrix-array photodetector (16) arranged to acquire an image of the sample, on being exposed to a light wave (22) resulting from interference between said incident light wave (12) and a diffraction wave formed by said cell;
- a holder, for holding the sample between said light source (11) and the matrix-array photodetector (16);
- a processor (20) configured to implement the following step:
- identifying a position ($x_n$,$y_n$) of said cell in a plane parallel to a plane in which the photodetector lies;

the device being **characterized in that** said processor (20) is configured to implement the following steps:

- applying a digital reconstruction algorithm to said acquired image, so as to determine at least one characteristic quantity ($u$, $\varphi$, $\tilde{u}$, $k$) of the light wave (22) to which the photodetector is exposed, at said position ($x_n$,$y_n$), at a plurality of what are called reconstruction distances ($|z|$) from said photodetector along the propagation axis (Z); and
- classifying said cell depending on a profile representing the variation in the characteristic quantity along the propagation axis, the classification being suitable for determining the state of the cell from among preset states.

14. Device according to Claim 13, wherein the device includes no magnifying optics between the matrix-array photodetector (16) and the analyzed sample (14).

15. Incubator, intended for the growth of cells, the incubator comprising at least one device according to Claims 13 and 14.

**Fig. 1**

**Fig. 2**

**Fig. 3A**          **Fig. 3B**          **Fig. 3C**

**Fig. 4A**

**Fig. 4B**

**Fig. 5A**

**Fig. 5B**

**Fig. 6A**

**Fig. 6B**

**Fig. 7**

Fig. 8A

Fig. 8B

Fig. 8C

Fig. 8D

Fig. 8E

Fig. 8F

**Fig. 9A**

**Fig. 9B**

**Fig. 9C**

**Fig. 9D**

**Fig. 9H**

**Fig. 9E**

**Fig. 9F**

**Fig. 9G**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008090330 A
- US 20120148141 A
- WO 2014012031 A

**Littérature non-brevet citée dans la description**

- **RYLE et al.** Digital in-line holography of biological specimens. *Proc. Of SPIE,* 2006, vol. 6311